# EUROPEAN PATENT APPLICATION

(11) **EP 1 348 386 A1**
(43) Date of publication of application: **01.10.2003**
(21) Application number: 03251977.9
(22) Date of filing: 28.03.2003
(51) Int. Cl.: A61B 17/34

(54) **Trocar with a reinforced seal**

(30) Priority: 29.03.2002 US 112184
(71) Applicant: ETHICON ENDO-SURGERY, INC., Cincinnati, Ohio 45245 (US)
(72) Inventor: Vitali, Dario, Cincinnati, Ohio 45215 (US)
(74) Representative: Mercer, Christopher Paul

(57) **Abstract**

A trocar (28) for performing a procedure on a patient. The trocar includes a hollow cannula (31) having a distal end (12) and a proximal end (14), and a housing (32) having a distal end (16) attached to the proximal end (14) of the cannula (31) and a proximal end having a wall attached thereto. The wall has an aperture (35) therethrough. The trocar further includes a first seal (26) attached to the housing for sealing around instruments passing through the aperture. The seal is an elastomeric member having an opening therethrough. The elastomeric member has metal reinforcing members disposed therein which are preferably made from a superelastic material such as Nitinol.

## Description

### Field of the Invention

The present invention relates, in general, to surgical trocars for providing access to a surgical site during endoscopic surgery. More particularly, the invention relates to a trocar having an improved seal to prevent tearing, which can lead to leakage of insufflation fluid from a surgical site through the trocar when an instrument is inserted through the trocar.

The present invention has application in conventional endoscopic and open surgical instrumentation as well application in robotic-assisted surgery.

### Background of the Invention

The use of endoscopic procedures in surgery has become widely accepted. The term endoscopic as used herein is defined to include all types of minimally invasive surgical procedures including laparoscopic and arthroscopic procedures. Accordingly, numerous endoscopic instruments have been developed which allow the surgeon to perform complex surgical procedures with minimal incisions into the skin and tissue surrounding a particular body cavity or anatomical region. In order to introduce the endoscopic instrumentation into the body cavity, it is often necessary to puncture and cannulate the body cavity by using a trocar. Trocars are widely known in the art and typically consist of an obturator and a trocar cannula. An example of a trocar can be found in U.S. Patent 6,017,356 issued to Frederick et al. on January 25, 2000, which is hereby incorporated herein by reference.

It is common for a sealing arrangement or sealing device to be used in association with the cannula to prevent the escape of fluid or gas during endoscopic procedures. During an endoscopic surgical procedure, the internal gas pressure must be maintained in order to successfully complete the procedure. In order to maintain the internal gas pressure while instruments are passed into and out of the trocars positioned in a body cavity, sealing devices are required for both the instruments and for the trocar assemblies. That is most trocars have two sealing devices. One which seals the trocar when there is not an instrument passing therethrough, and one which seals the trocar as instruments are passed therethrough. Furthermore, it is desirable that the sealing device maintain gas pressure in the abdominal cavity, despite numerous insertions and withdrawals of surgical instruments through the trocar cannula.

Most commercially available trocars have an outer seal and an inner seal. The outer seal is typically a gasket located at the proximal most end of the trocar cannula. This gasket tightly fits itself around the elongated shafts of any medical devices passing therethrough. Therefore, the outer seal prevents fluids from escaping the body cavity through the trocar cannula while surgical instruments are being used with the cannula. The inner seal is typically what is referred to as a flapper door. It is made from a rigid, typically plastic, door which is spring biased against an inner gasket. The inner seal prevents fluids from escaping the body cavity through the trocar cannula while the trocar cannula is not in use, i.e. with no surgical instruments or obturators passing therethrough. The inner seal is located with the trocar cannula handle, distal to the outer seal.

While for the most part the above design works well, there has been a tendency for the outer seal, or gasket, to tear during surgery. If a physician were to align a surgical instrument directly over the outer gaskets opening, with the longitudinal axis of the instrument parallel with the longitudinal axis of the trocar cannula, tearing would not be an issue. However, often times the physician enters the trocar cannula with the surgical instrument at an angle with respect to the cannula. This often causes the tip of the surgical instrument to be forced upon the wall of the gasket causing the gasket to tear. A torn gasket can lead to leaks during the surgical procedure, and may require the physician to insert a new cannula.

Others have tried to solve this problem. One attempted solution was a floating gasket, wherein the outer gasket floated on a bellows type structure. Examples of this type of seal are given in U.S. Patents 5,209,737 issued to Ritchart et al. on May 11, 1993; 5,385,553 issued to Hart et al. on January 31, 1995; and 5,308,336 issued to Hart et al. on May 3 1994, all of which are hereby incorporated herein by reference. However, this type of floating seal does not always significantly reduce the incidents of tearing. In addition, these types of seals can often be expensive.

Another attempt is a gasket molded of two layers of different hardness. A softer inner layer to form the seal and a harder outer layer to protect the seal from the abrasion of the operating instrument. An example of this type of seal is found in U.S. Patent 5,628,732 issued to Antoon, Jr. et al. on May 13, 1997. Yet, another attempt is a seal fabricated so that the outer surface of the seal is protected from abrasion by an iris type component; an example of the is found in U.S. Patent 5,308,336 issued to Hart et al. on May 3, 1994. And yet, another attempt is to provide a spandex fabric layer around the elastomeric seal or to envelop the fabric with the seal to prevent tearing of the seal. And example of this method is European Patent 1015049 (WO98/53865) published on December 3, 1998. However, all of these attempts had their own shortcomings.

Therefore, there has been a desire for an improved trocar seal design which significantly reduces the incidents of tearing during surgical procedures.

### Summary of the Invention

A trocar for performing a procedure on a patient. The trocar includes a hollow cannula having a distal end and a proximal end, and a housing having a distal end attached to the proximal end of the cannula and a proximal end having a wall attached thereto. The wall has an aperture therethrough. The trocar further includes a first seal attached to the housing for sealing around instruments passing through the aperture. The seal is an elastomeric member having an opening therethrough. The elastomeric member has metal reinforcing members disposed therein which are preferably made from a superelastic material such as Nitinol.

### Brief Description of the Drawings

The novel features of the invention are set forth with particularity in the appended claims. The invention itself, however, both as to organization and methods of operation, together with further objects and advantages thereof, may best be understood by reference to the following description, taken in conjunction with the accompanying drawings in which:
FIG. 1 is an isometric view of a trocar inserted through a body wall, providing access for a surgical instrument within a body cavity, where a seal is incorporated into the trocar;
FIG. 2 is an exploded isometric view of the proximal end of a cannula housing and the seal of the trocar illustrated in FIG. 1;
FIG. 3 is an isometric view of the seal;
FIG. 4 is a plan view of a reinforcing element contained within the seal shown in FIG. 3;
FIG. 5 is a plan view of the seal illustrated in FIG. 3, shown in partial cross-section; and
FIG. 6 is a schematic top plan view of the proximal end of the cannula housing showing the seal sealing against a surgical instrument as seen along view line 6--6 of FIG. 2.

### Detailed Description of the Invention

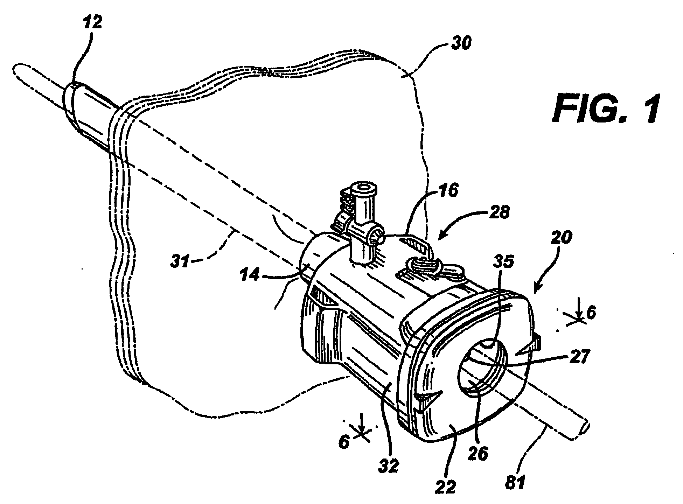

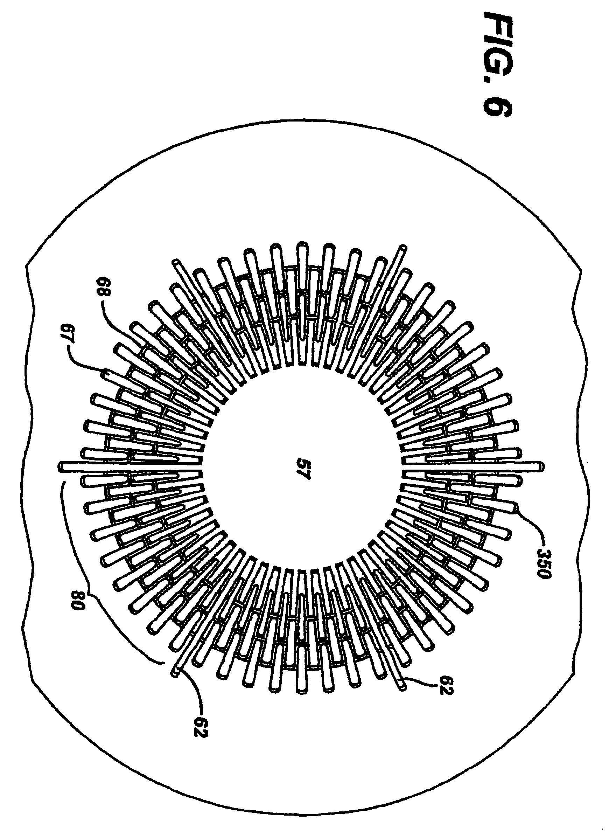

Referring to the drawings wherein like numerals indicate the same element throughout the views, there is shown in Figure 1 a trocar 28 made in accordance with the present invention. Trocar 28 provides access for the insertion of a surgical instrument 81, such as a clip applier, stapler, grasper, cutter, etc., into a body cavity. Trocar 28 comprises a cannula 31 and an obturator (not shown). Cannula 31 has a distal end 12 and a proximal end 14. Trocar 28 further includes a housing 32 having a distal end 16 attached to the proximal end 14 of cannula 31. Housing 32 has a proximal end 18, having an aperture 35 therein. FIG. 1 shows the trocar 28 after it has penetrated a body wall 30 of a surgical patient, and the obturator has been subsequently withdrawn from the cannula 31. Housing 32 has an interior chamber 33 (see FIGS. 6), communicating with the interior of cannula 31. Distal end 12 of cannula 31 is shown extending through an opening made in the body wall 30. Trocar 28 includes a first seal 26 attached to said housing and sealing said aperture 35. Aperture 35 has a centrally located opening 27 extending therethrough so that the seal seals around interment 81 as it is passed therethrough.

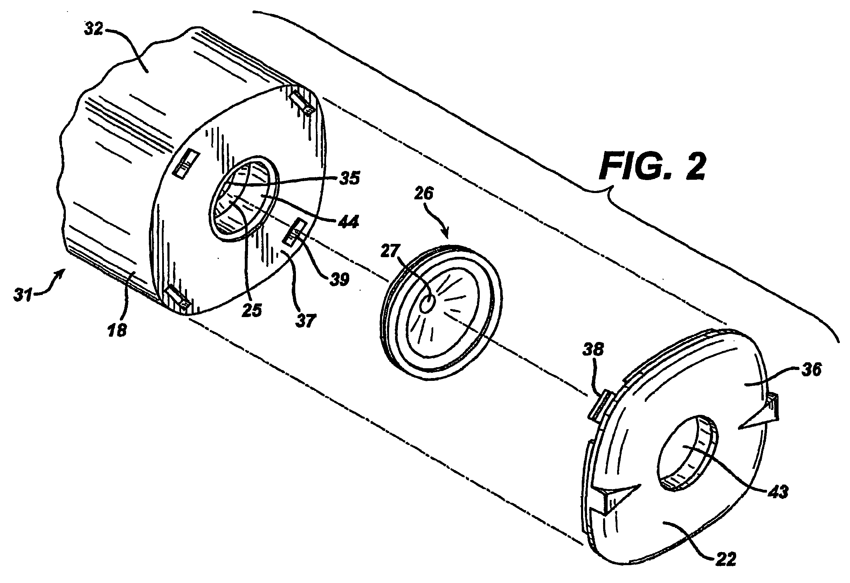

The seal 26 can be better understood by referring to FIG. 2. As seen therein, the seal 26 can affixed to the housing by being captured between the proximal end 18 of the housing and a frame 36. The frame 36 has projections 38 which snap fit into receiving slots 39 in order to retain the frame onto the housing.

The seal 26 includes the opening 27 for the passage of the surgical instrument 81. Opening 27 elastically expands to allow passage of surgical instruments 81 inserted therein. The opening 27 can have a diameter between about 2.5 to about 5 mm. Accordingly, the seal 26 should have the dual properties of elasticity to enable the passage of large diameter surgical instruments therethrough, and toughness to prevent ripping when surgical instruments 81 are passed through the seal 26.

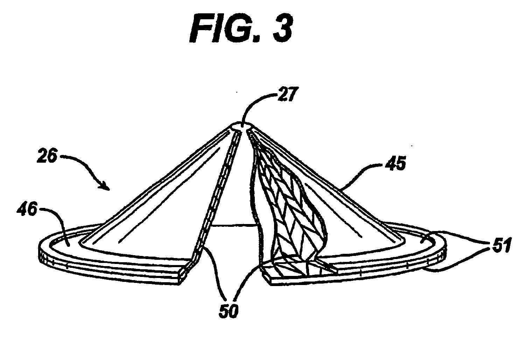

FIG. 3 shows one embodiment of the seal 26 made in accordance with the present invention. The seal 26 may comprise a medical grade elastomeric material 51 encapsulating a reinforcing member 50. The seal 26 can be of any shape such as a disk or cone and, as illustrated, can have a conical inner portion 45 and a disk shaped outer portion 46. Examples of suitable elastomeric materials for 51 include, but are not limited to, silicone rubber, polyurethane elastomer, chlorobutyl rubber, latex rubber, polyisoprene rubber and ethylene propylene diene monomer ("EPDM") rubber medical grade silicone, for example, Dow Corning SLASTIC™ brand medical grade silicone rubber.

The reinforcing member 50 can also be elastically deformed along with the seal 26 and is shown in its original unconstrained state in FIG. 3. The reinforcing member 50 can be constructed of a metal which is capable of resilient deflection, such as an elastic or spring grade of steel, stainless steel, copper or a titanium alloy. The reinforcing member 50 can also be made from a pseudoelastic or superelastic material such the nickel titanium alloy NITINOL™ from Raychem Corp., Menlo Park, Calif. The reinforcing member 50 can be made in the shape memory alloy's super elastic phase which can withstand very large deflections without deformation. When the reinforcing member 50 is distorted or expanded, it will return to its original shape when released. The reinforcing member 50 can be woven from a filament or formed or stamped from a sheet or foil of metal.

When member 50 is made from NITINOL alloy, the alloy preferably comprises from about 50.5% (as used herein these percentages refer to atomic percentages) Ni to about 60% Ni, and most preferably about 55% Ni, with the remainder of the alloy Ti. Preferably, the reinforcing member 50 is such that it is superelastic at body temperature, and preferably has an A_{f}, the temperature at which the alloy transforms completely from the martensite phase to the austenite phase, in the range from about 24°C to about 37°C. As mentioned above, it is preferred that the reinforcing member 50 be made from a superelastic alloy and most preferably made of an alloy material having greater than 50.5 atomic % Nickel and the balance titanium. Greater than 50.5 atomic % Nickel allows for an alloy in which the temperature at which the martensite phase transforms completely to the austenite phase, the A_{f} temperature, is below human body temperature and preferably is about 24°C to about 37°C so that austenite is the only stable phase at body temperature. A suitable shape memory alloy is available as NITINOL™ from Raychem Corp., Menlo Park, Calif.

Reinforcing member 50 can be composed of a mesh design where the filaments or linear components of formed design form included angles ranging from five to 175 degrees. Alternatively, reinforcing member 50 can be composed of a mesh design containing curved filaments or etched design components. The angled or curved configuration of the filaments or legs of the reinforcing member 50 allows radial expansion to facilitate insertion of surgical instruments 81 of various diameters. Designs for reinforcing member 50 which do not exhibit substantial change in axial length upon radial expansion or contraction can be desirable in a conical seal.

Seal 26 can be assembled by casting, by injection molding the elastomeric material 51 around the reinforcing member 50 or by compression molding. The open structure of the reinforcing member 50 can allow the elastomeric material 51 to flow through and surround the reinforcing member 50 during the molding process. Laminated elastomeric layers attached to the reinforcing member 50 or other manufacturing techniques which are known in the art may also be used.

The reinforcing member 50 constrained within the elastomeric material 51 is designed to prevent the tearing of the elastomeric material 51 and to prevent the propagation of tears or cracks in the elastomeric material 51 during use. If the surgical instrument 81 is inserted out of alignment with the opening 27 and contacts the inner portion 45 of the seal 26, penetration of the elastomeric material 51 is limited by the size of the openings in the reinforcing member 50. This limited penetration limits the egress of insufflation fluids through the penetration. Additionally, the adhesion of the elastomeric material 51 to the mesh of the reinforcing member 50 can prevent the spreading of the penetration to adjacent openings in the mesh.

The reinforcing member 50 can includes an opening aligned with opening 27 which larger than the opening 27. Thus, the innermost area of the seal 26 can be formed solely from elastomeric material to provide a more uniform seal against the surgical instrument 82.

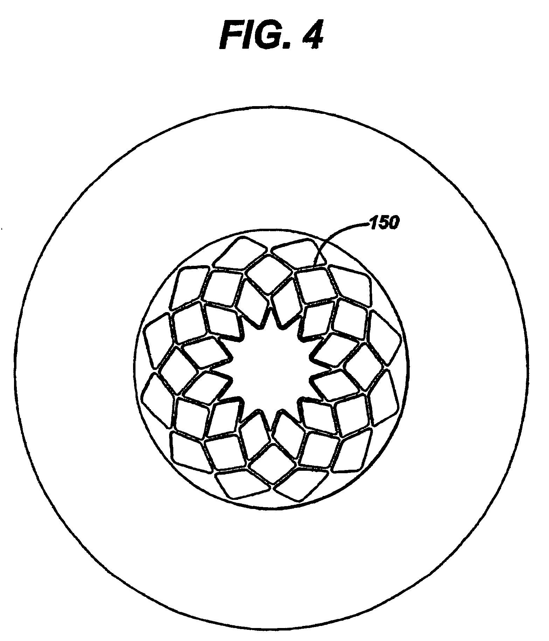

FIG. 4 shows an alternative embodiment 150 of the reinforcing member. The angles, spacing and patterns are etched into member 150 such that they form a series of diamond shapes having members or legs which join at angles between five and 175 degrees. The included angles vary as a function of radial position to keep area between enclosing members approximately equal.

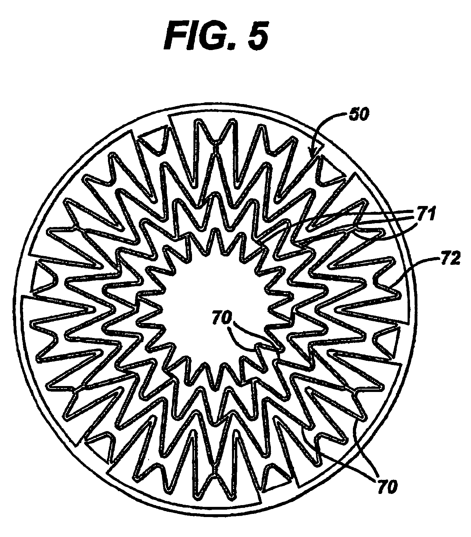

A second alternate embodiment of a reinforcing member 250 is shown in FIG. 5. Here the reinforcing member depicts concentric rings of angular star patterns 70, periodically connected by linking members 71 to form a matrix. The radial expansion of the star pattern may be accommodated by the periodic inclusion of irregular elements 72.

Figures 6 depict yet another embodiment of a reinforcing member 350. Member 350 is arranged as a matrix of flat members 67 interconnected by struts 68. The entire member 350 is interrupted by slots 62 which divide member 350 into several leafs 80. Having these slots provide for a member which will more easily accept a large diameter surgical instrument while uniformly stretching the elastomeric material surrounding it.

These embodiments are only representative of several possible patterns and are not intended to represent all possible patterns applicable to the present invention. It should be obvious to one skilled in the art that the reinforcing member can take on many different patterns or configurations that would appropriately protect the seal 26 from tearing. Other configurations and methods of manufacture in addition to those described above are known.

While preferred embodiments have been shown and described herein, it will be obvious to those skilled in the art that such embodiments are provided by way of example only. Numerous variations, changes, and substitutions will now occur to those skilled in the art without departing from the invention. In addition, any structure herein can be described as a means for performing its associated function. Accordingly, it is intended that the invention be limited only by the spirit and scope of the appended claims.

## Claims

1. A trocar for performing a procedure on a patient, said trocar comprising:
a. a hollow cannula having a distal end and a proximal end;
b. a housing having a distal end attached to said proximal end of said cannula and a proximal end having a wall attached thereto, said wall having an aperture therethrough;
c. a first seal attached to said housing for sealing around instruments going through said aperture, said seal comprising an elastomeric member having an opening therethrough, said elastomeric member having reinforcing members disposed therein, wherein said reinforcing members comprise metal.

2. The trocar of claim 1 further comprising a second seal, within said housing and distal to said aperture, said second seal having an open position and a closed position, wherein when said second seal is in said open position there is fluid communication between said aperture and said cannula, and wherein when said second seal is in said closed position, fluid communication between said aperture and said cannula is substantially prevented.

3. The trocar of claim 1 wherein said second seal comprises of a flapper spring biased against a gasket.

4. The trocar of claim 1 wherein said reinforcing members comprise a superelastic alloy.

5. A trocar for performing a procedure on a patient, said trocar comprising:
a. a hollow cannula having a distal end and a proximal end;
b. a housing having a distal end attached to said proximal end of said cannula and a proximal end having a wall attached thereto, said wall having an aperture therethrough;
c. a first seal attached to said housing for sealing around instruments going through said aperture, said seal comprising an elastomeric member having an opening therethrough, said elastomeric member having reinforcing members embedded within, wherein said reinforcing members comprising a superelastic alloy metal.

6. The trocar of claim 5 further comprising a second seal, within said housing and distal to said aperture, said second seal having an open position and a closed position, wherein when said second seal is in said open position there is fluid communication between said aperture and said cannula, and wherein when said second seal is in said closed position, fluid communication between said aperture and said cannula is substantially prevented.

7. The trocar of claim 5 wherein said second seal comprises of a flapper spring biased against a gasket.

8. The trocar of claim 1 or claim 5 wherein said reinforcing members comprise NITINOL.

9. The trocar of claim 1 or claim 5 wherein said first seal is comprised of silicone

10. The trocar of claim 1 or claim 5 wherein said first seal is comprised of polyisoprene.
